(19) Europäisches Patentamt
European Patent Office
Office européen des brevets

(11) Publication number: **0 090 868**
**A1**

(12) **EUROPEAN PATENT APPLICATION**

(21) Application number: 82102889.1

(22) Date of filing: 05.04.82

(51) Int. Cl.³: G 01 N 33/72
G 01 N 31/08

(43) Date of publication of application: 12.10.83 Bulletin 83/41

(84) Designated Contracting States: AT BE CH DE FR GB LI NL SE

(71) Applicant: Miles Italiana SpA
Via FL Miles 10
I-20040 Cavenago Brianza (Mi)(IT)

(72) Inventor: Berti, Giovanni
Via Provinciale 34
I-22040 Malgrate (Como)(IT)

(72) Inventor: Heyda, Alessandro
Viale Zara 119
20159 Milano(IT)

(74) Representative: Senftl, Hannes, Dr.
c/o Bayer AG Zentralbereich Patente Marken und Lizenzen
D-5090 Leverkusen 1, Bayerwerk(DE)

(54) **Test for glycosilated hemoglobins.**

(57) The invention provides an improved process for the ion-exchange chromatographic separation of compounds, such as hemoglobins, by passing a separator composition, which includes an ampholyte through the chromatography system prior to the introduction of the sample. When the assay is carried out using the separator composition as described in the present invention, the separation between $HbA_1$ and HbA is improved such that the results obtained are independent of the temperature in a large range, i.e., at least from 15 to 35°C. The invention also provides a test kit.



Croydon Printing Company Ltd.

# TEST FOR GLYCOSILATED HEMOGLOBINS

## *BACKGROUND OF THE INVENTION*

### *Field of the Invention*

The present invention relates to the field of diagnostic test compositions and methods, such as are useful in manual and automated systems, and, more particularly, to those tests useful in qualitative and quantitative determination of glycosilated hemoglobins in a liquid sample.

### *Description Of The Prior Art*

Glycosilated hemoglobins ($HbA_1$) are modified forms of normal adult hemoglobin (HbA) having molecules which contain four polypeptide chains, two α and two β, thus being also identified as $\alpha_2\beta_2$. $HbA_1$ is composed of at least three minor components: $HbA_{1a}$, $HbA_{1b}$ and $HbA_{1c}$. All three fractions have the same amino acid sequence, but appear to contain a different carbohydrate.

Hemoglobin $A_{1c}$ is the major component of $HbA_1$: it represents approximately 85% of glycosilated hemoglobins. The $HbA_1$ molecule has the terminal amino group of the β chains bound to a molecule of glucose. This molecule is formed, *in vivo*, by the following reaction:

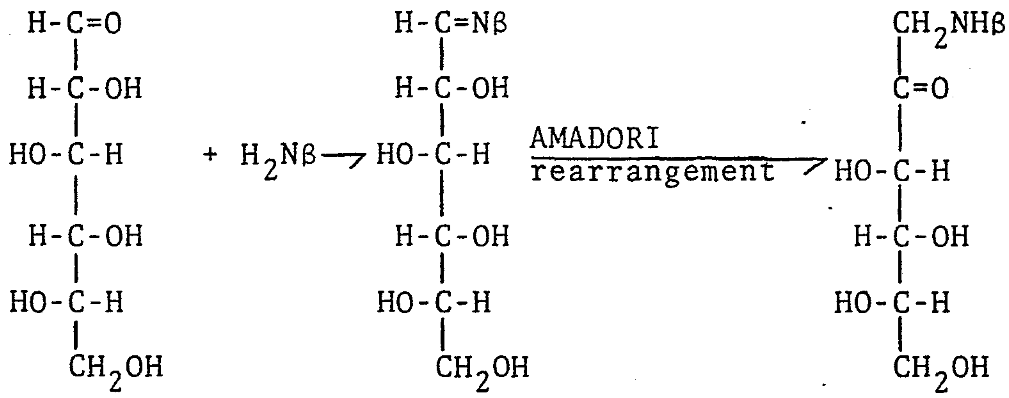


glucose βchain aldimine form ketoamine form

Glycosilated hemoglobin levels, in normal adult blood, range from 3% to 6% of total hemoglobin, but can rise as high as 15% in uncontrolled diabetes mellitus, because $HbA_{1c}$ levels double or triple.

The strict relation of $HbA_{1c}$ concentration to diabetic control has been demonstrated by several authors [see, e.g. H.F. Bunn *et al.*, Science, 200, 21-27 (1978)].

If a diabetic patient is in "poor" diabetic control, he will often have high levels of glucose and glycolytic intermediates in plasma and, consequently, within the red cells because they do not require insulin for glucose uptake.

This rise in intracellular glucose stimulates the non-enzymatic glycosilation of HbA. In these conditions, the stability of the covalent linkage between the hexose and the protein, together with

the relatively long life of the hemoglobin molecule leads to an increased level of $HbA_1$. Consequently, hemoglobin $A_1$ concentration reflects the mean blood sugar level over the previous weeks.

The periodic monitoring of $HbA_1$ values can provide an objective and accurate estimate of the degree of carbohydrate control in diabetic patients, and allows the physician to evaluate the effectiveness of the therapy.

Different methods have been developed for the determination of glycosilated hemoglobins (also called fast hemoglobins because of their chromatographic behavior). These methods are based on colorimetry, isoelectric focusing and chromatography.

In the colorimetric method of Flückiger [R. Flückiger and K.H. Winterhalter, FEBS Letters, 71, 356 (1976)], the hemolysate is treated with 0.4 N oxalic acid at 100° Centigrade (C). Under these conditions, HbA, quantitatively releases 5-hydroxy methylfurfural (5-HMF), which can be detected by the color developed with 2-thiobarbituric acid (reading at 443 nanometers). This method has many disadvantages, such as the high temperature (100°C) and long time (4 hours) required for assaying a sample and the poor reproducibility (due to the low stability of 5-HMF) and low specificity (free sugars also react). It is considered unsuitable for routine work.

Recently, P.G. Righetti *et al.*, [American J. of Hematology, 4, 367-374 (1978)] proposed a modified type of isoelectric focusing, in slabs of polyacrylamide gel, for evaluating hemoglobin $A_1$. In this method a 600 nanometer (nm) scan gives two completely resolved peaks, which can be integrated accurately

for quantitative determinations. The method is claimed to be highly specific and reproducible, but requires expensive reagents such as ampholines, and expensive instruments (the gel should be scanned by a spectrophotometer with a slit of 100 nm and equipped with a linear transport apparatus, and the areas under both peaks integrated by a minicomputer). This procedure, therefore, could be considered as a good comparison method, but it is not within the financial reach of a medium laboratory.

Although some work had been performed earlier, the ion-exchange chromatography methods now used are based on Trivelli's original procedure [L. Trivelli *et al.*, N. Eng. J. Medicine, 284, 353 (1971)]. In this procedure, a dialyzed blood hemolysate is added to a 2 centimeter (cm) x 17.5 cm column, packed with a weakly acidic cation exchange resin (Bio-Rex® 70). $HbA_{1a}$, $HbA_{1b}$ and $HbA_{1c}$ are eluted together with a sodium phosphate buffer (42 millimolar phosphate pH 6.70) containing cyanide (10 millimolar KCN). After subsequent elution of HbA with a more concentrated developer (150 millimolar phosphate, pH 6.42), $HbA_1$ percentage can be calculated as the ratio between the absorbance values of the two eluates (reading at 552 nm). This method is considered sufficiently reliable, but requires several days for the equilibration of the resin with developer, several hours for the dialysis of the hemolysate at 4°C, and three hours of elution time. For all these reasons, this procedure is unsuitable for routine clinical application.

Attempts have been made to provide products which simplify this expensive and time-consuming method. Kits are now available on the market from Isolab Inc. (Drawer 4350 Akron, Ohio), Bio-Rad Laboratories (2200 Wright Ave., Richmond, Calif.) and Helena Laboratories (P.O. Box 752, Beaumont, Texas) with which the $HbA_1$ chromatographic determination can be carried out in less time and at less cost than was possible with the original Trivelli method. In these commercial products, the principle still consists in the elution of the sample from a cationic ion-exchange resin, using a developer which is generally a phosphate buffer and which also may contain potassium cyanide. The columns are very small in comparison with those of the original Trivelli method, the sample is consequently smaller, and they are prefilled with a resin already equilibrated with an appropriate buffer. Hemolysates are obtained very quickly from whole blood, utilizing a suitable lysing reagent. The elution takes about half an hour or an hour depending whether HbA is also eluted (in a second step) or not.

The most common analytical procedure (simplified) which is used in routine clinical chemistry, therefore consists of the following steps:

(1) A whole blood sample is hemolysed;

(2) a small amount of hemolysate (e.g. 50 or 100 µl) is applied to the top of a chromatography column which contains a weak cationic resin equilibrated with an appropriate buffer;

(3) the fast-moving fraction of hemoglobin ($HbA_1$) is eluted with a sodium phosphate buffer which may also contain potassium cyanide; and

(4) then, the slow-moving fraction (HbA) is eluted with a more concentrated elution buffer containing sodium phosphate and sodium chloride, which may contain potassium cyanide.

The eluates are read in a spectrophotometer, e.g. at 415 nm against distilled water or buffer. The percentage of fast hemoglobin (Hbs%) is calculated by the following formula:

$$\frac{A_{415}\ HbA_1}{A_{415}\ HbA_1 + A_{415}\ HbA} \times 100 = Hbs\%$$

Alternatively, instead of collecting the slow fraction too, an aliquot of the hemolysate can be diluted with an appropriate volume of the elution buffer. In this case, both the eluate ($HbA_1$) and the above described solution (total Hb) are read in the spectrophotometer against distilled water. Hbs% is calculated by the following formula:

$$\frac{A_{415}\ HbA_1}{A_{415}\ total\ Hb} \times 100 = Hbs\%$$

These commercial products have provided a way to assay glycosilated hemoglobins under routine clinical chemistry, but they have been subjected to criticism because of their poor reproducibility and accuracy. A check of one of the commercial products based on this method has shown that the percentage of "fast hemoglobin" found increases linearly from 10°C to 37°C [D. Dixon *et al.*, Clin. Chem. 24, 2073 (1978)]. This can be ascribed to an incomplete separation between the two peaks ($HbA_1$ and HbA), which become closer and closer as the ambient temperature increases.

## *SUMMARY OF THE INVENTION*

The invention provides an improved procedure for the ion-exchange chromatographic separation of compounds, such as hemoglobins, by passing a separator composition, which includes an ampholyte, as described more fully below, through the chromatography system prior to the introduction of the sample to be chromatographed. The separator spaces the elution peaks or prevents undesirable elutions, making the determination of the eluted component(s) more accurate. When the assay is carried out using the separator composition as described in the present invention, the separation between $HbA_1$ and HbA is improved such that the results obtained are independent of the temperature in a large range, i.e. at least from 15 to 35°C.

In accordance with the present invention there is, therefore, provided a method for the chromatographic determinaton of compounds, such as glycosilated hemoglobins. In one embodiment, the method according to the invention for the chromatographic determination of glycosilated hemoglobins is of the type having the steps of:

(1) applying a hemoglobin-containing blood hemolysate to a column of ion exchange material suitable for the chromatographic separation thereof;

(2) eluting a first fraction of the hemolysate, containing the glycosilated hemoglobins, with a developer solution;

(3) collecting the first fraction so eluted;

(4) eluting a second fraction, containing the slower moving hemoglobins, with the same or a different developer solution;

(5) collecting the second fraction so eluted;

(6) measuring the amount of glycosilated hemoglobin present in the first fraction; and

(7) measuring the amount of slower moving hemoglobin present in the second fraction so that the percentage of total hemoglobin which is glycosilated hemoglobin can be determined, having improvement wherein;

(8) prior to applying the hemoglobin-containing blood hemolysate to the column, the method comprises the additional step of applying a solution of a separator composition which includes at least one ampholyte to said column.

In another embodiment, the method according to the invention for the determination of glycosilated hemoglobins is of the type having the steps of:

(1) applying a hemoglobin-containing blood hemolysate to a column of ion exchange material suitable for the chromatographic separation thereof;

(2) eluting a fraction of the hemolysate, containing the glycosilated hemoglobins, with a developer solution;

(3) diluting an aliquot of the hemolysate with an appropriate volume of the elution developer;

(4) measuring the amount of glycosilated hemoglobin present in the eluted fraction; and

(5) measuring the amount of total hemoglobin in the diluted aliquot so that the amount of total hemoglobin which is glycosilated hemoglobin can be determined, having the improvement wherein;

(6) prior to applying the hemoglobin-containing blood hemolysate to the column, the method comprises the additional step of applying a solution of a separator composition which includes at least one ampholyte to said column.

The invention also provides a test kit to easily and quickly perform an accurate assay of glycosilated hemoglobins under routine conditions. For this purpose, the kit contains, in packaged combination, at least one chromatography column pre-filled with an ion exchange material suitable for the chromatographic separation of a hemoglobin-containing blood hemolysate, such as a weakly acidic cation exchange resin, and at least one container each of an eluting developer and a separator component, which can be in the form of a powder to be reconstituted or a ready-to-use solution. Optionally, there may also be containers of a lysing solution and a control having a known percentage of glycosilated hemoglobins.

## DESCRIPTION OF THE PREFERRED EMBODIMENTS

Although specific terms are used in the following description for the sake of clarity, these terms are intended to refer only to the particular embodiments of the invention selected for illustration and are not intended to define or limit the scope of the invention.

The term "ampholyte" as used herein is contemplated to include any product containing in its molecule at least one acidic and at least one alkaline group, whatever its kind may be. The acidic group(s) will preferably be a weak acid, e.g. one containing a carboxyl group, and the basic group(s) will preferably be a weak base, e.g. one containing an amino group. In particular, amino acids and polypeptides such as glycine, alanine, valine, leucine, glycylglycine or their mixtures are particularly suitable. In case of amino acids or polypeptides, products which have an isoelectric point ranging from about 4 to about 9, preferably from about 5.5 to about 6.5, may be used. The pH of the amino group may vary from about 7 to about 11, but is preferably between about 9 and about 10. The number of carbon atoms may be from 2 up, the upper limit being fixed only by the solubility of the product, and preferably it will be from about 2 to about 6.

The dipolar ion is preferably dissolved in water, in a concentration ranging from 20 mM/liter up to the solubility limit, preferably from 0.2 M/liter upwards. A volume of separator solution containing this ampholyte is added to the top of a chromatographic column pre-filled with a weakly

acidic cationic resin previously equilibrated according to the prior art, and the column is allowed to drain until the separator is completely eluted.

This eluate is discarded and the pre-treated columns are utilized according to the usual procedures for chromatographic $HbA_1$ evaluation.

The examples now provided are illustrative of the invention. One skilled in the art will be able to make such variations, substitutions and changes as may seem desirable.

EXAMPLE I

In the experiment described by this example, a chromatographic column was prepared and tested for its ability to quantitatively determine the presence of glycosilated hemoglobins in a liquid sample.

Column Preparation

Plastic columns having an 11 millimeter (mm) internal diameter and a 68 mm height were filled with about 800 milligrams (mg) of the cationic resin Bio-Rex® 70 (Bio-Rad), sodium form, 200-400 mesh, previously equilibrated with 50 mM sodium phosphate buffer, pH 6.8.

Analytical Procedure

One group of the columns was used without any further treatment, according to the prior art, and the remaining columns were treated with 1.0 milliliter (ml) of a 15 grams/ deciliter (g/dl) glycine solution, according to the present invention, by adding this solution on the top of the column and allowing it pass through the resin.

Then, 100 microliters (µl) of hemolysate (one volume of blood + 15 volumes of a Brij® 35 solution, 0.33%) was added to the top of each series of columns. The fast fraction was eluted with 2.5 ml of sodium phosphate buffer, pH 6.8, containing potassium cyanide (50 mM phosphate, 7 mM KCN). A solution containing all the hemoglobins was also prepared (20 µl of hemolysate + 5 ml of buffer). Both the eluate

and the above described solution were read in a spectrophotometer at 415 nm, against distilled water. The $HbA_1$ percentage was calculated by the following formula:

$$\frac{A_{415}\ \text{fast fraction}}{A_{415}\ \text{total Hbs}} \times 10 = HbA_1\%$$

The tests were performed with both series of columns (untreated and treated with glycine solution) at temperatures ranging from 15 to 35°C.

Results

The percent of fast hemoglobins found in samples of blood processed at different temperatures are reported in Table 1:

TABLE 1

| °C | Present Invention | Prior Art |
|---|---|---|
| 15 | 5.38 | 4.44 |
| 20 | 5.43 | 5.38 |
| 25 | 5.39 | 7.35 |
| 30 | 5.45 | 9.42 |
| 35 | 5.90 | 15.10 |

Conclusion

The data of Table 1 show that the results obtained with the procedure according to the present invention are practically unaffected by temperature.

## EXAMPLE II

The experiments reported by this example were performed by the same procedure as those of Example I. The exceptions are that a different resin, developer and ampholyte were used.

### Element Preparation

Small plastic columns (4 mm internal diameter, 150 mm height) were filled with about 150 mg of the cationic resin Amberlite® CG 50 (Rohm & Haas), type II, previously equilibrated with 30 mM sodium phosphate buffer, pH 6.7.

### Analytical Procedure

As in Example I, part of the columns were used without any further treatment and part were treated with 2.0 ml of 8 g/dl alanine solution according to the present invention.

Tests on a sample of hemolysate were performed in both series of columns as described in Example 1, except that the developer was in this case 30 mM phosphate buffer containing 7 mM sodium azide.

### Results

The percent of fast hemoglobins found in samples processed at different temperatures are reported in Table 2:

TABLE 2

| °C | Present Invention | Prior Art |
|---|---|---|
| 15 | 10.40 | 9.18 |
| 20 | 10.75 | 10.84 |
| 25 | 10.82 | 13.36 |
| 30 | 10.96 | 16.10 |
| 35 | 11.66 | 19.79 |

Conclusion

The data of Table 2 show once again the temperature independence of results obtained by the procedure according to the present invention and the high variability of results according to ambient temperature when a procedure according to the previous art is used.

Although it has been described with particularity, numerous changes in the details of the invention can be resorted to without departing from the scope of the invention.

WHAT IS CLAIMED IS:

1. A process for the chromatographic determination of glycosilated hemoglobins comprising the steps of:

(1) applying a hemoglobin-containing blood hemolysate to a column of ion exchange material suitable for the chromatographic separation thereof;

(2) eluting a first fraction of the hemolysate, containing the glycosilated hemoglobins, with a developer solution;

(3) collecting the first fraction so eluted;

(4) eluting a second fraction, containing the slower moving hemoglobins, with the same or a different developer solution;

(5) collecting the second fraction so eluted;

(6) measuring the amount of glycosilated hemoglobin present in the first fraction; and

(7) measuring the amount of slower moving hemoglobin present in the second fraction so that the percentage of total hemoglobin which is glycosilated hemoglobin can be determined, having the improvement wherein;

(8) prior to applying the hemoglobin-containing blood hemolysate to the column, the method comprises the additional step of applying a solution of a separator composition which includes at least one ampholyte to said column.

2. A process for the chromatographic determination of glycosilated hemoglobins of the type having the steps of:

(1) applying a hemoglobin-containing blood hemolysate to a column of ion exchange material suitable for the chromatographic separation thereof;

(2) eluting a fraction of the hemolysate, containing the glycosilated hemoglobins, with a developer solution;

(3) diluting an aliquot of the hemolysate with an appropriate volume of the elution developer;

(4) measuring the amount of glycosilated hemoglobin present in the eluted fraction; and

(5) measuring the amount of total hemoglobin in the diluted aliquot so that the amount can be determined, having the improvement wherein;

(6) prior to applying the hemoglobin-containing blood hemolysate to the column, the method comprises the additional step of applying a solution of a separator composition which includes at least one ampholyte to said column.

3. The process of any of claims 1 and 2 wherein the at least one ampholyte is of the type containing in its molecule at least one weakly acidic group.

4. The process of any of claims 1 and 2 wherein the at least one ampholyte is of the type containing in its molecule at least one weakly basic group.

5. The process of any of claims 1 and 2 wherein the at least one ampholyte included in said separator composition is at least one amino acid.

6. The process of any of claims 1 and 2 wherein the at least one ampholyte included in said separator composition is the combination of at least one amino acid and at least one polypeptide.

7. A test kit for the chromatographic determination of glycosilated hemoglobins which, in packaged combination, comprising at least one chromatography column pre-filled with an ion-exchange material suitable for the chromatographic separation of a hemoglobin-containing blood hemolysate and at least one container each of an eluting developer and a separator composition which includes at least one ampholyte.

8. The test kit of claim 7 wherein the at least one ampholyte is of the type containing in its molecule at least one weakly acidic group.

9. The test kit of claim 7 wherein the at least one ampholyte is of the type containing in its molecule at least one weakly basic group.

10. The test kit of claim 7 wherein the at least one ampholyte included in said separator composition is at least one amino acid.

European Patent Office

# EUROPEAN SEARCH REPORT

Application number
0090868
EP 82102889.1

## DOCUMENTS CONSIDERED TO BE RELEVANT

| Category | Citation of document with indication, where appropriate, of relevant passages | Relevant to claim |
|---|---|---|
| A | US - A - 4 270 921 (GRAAS) | |
| | * Claim 25 * | 1,2 |
| | * Claim 1 * | 7 |
| | ---- | |

CLASSIFICATION OF THE APPLICATION (Int. Cl. 3)

G 01 N 33/72
G 01 N 31/08

TECHNICAL FIELDS SEARCHED (Int.Cl. 3)

G 01 N

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| VIENNA | 05-11-1982 | SCHNASS |